# EUROPEAN PATENT APPLICATION

(11) **EP 0 988 855 A1**
(43) Date of publication of application: **29.03.2000**
(21) Application number: 99300566.9
(22) Date of filing: 26.01.1999
(51) Int. Cl.: A61K 7/48, A61K 7/00

(54) **Facial moisturizer and cleanser**

(30) Priority: 28.01.1998 US 14919
(71) Applicant: Kim, Hee Jung, Seoul 138-220 (KR)
(72) Inventor: KIM, Hee Jung, Songpa-ku, Seoul 138-220 (KR); SHIN, Myoung Soo, Seoul 136-020 (KR); PARK, Sang Chul, Seoul 137-044 (KR)
(74) Representative: Evens, Paul Jonathan

(57) **Abstract**

A cosmetic facial cleanser and moisturizer primarily composed of water subjected to electrolysis. This water is the product of a multi-step process comprising a plurality of separate batch processes, wherein each successive process yields a product with greater bactericidal and moisturizing effects than the product yielded from the previous process. The first process comprises the passage of an electric current through water without the presence of electrolytes, which yields water with a lowered viscosity. The resulting water is then placed into a series of batch energizer reactors, wherein the water is successively irradiated with far infrared light in each batch reactor, each successive reactor yielding a product with greater aforementioned effects. Receivers at each batch reactor allow the water to be properly drained from the reactors. Additional cleansing agents may be added.

## Description

### BACKGROUND OF THE INVENTION

### 1. Field of the Invention

The present invention pertains to beauty products, and specifically to products used to moisturize and cleanse the facial area.

### 2. Description of the Prior Art

The prior art pertaining to cosmetic facial cleansers include the following. U.S. Patent No. 5,658,573 to Holcomb discloses a method of generating an aqueous suspension of colloidal silica. U.S. Patent No. 4,140,656 to Mast discloses an anhydrous clear gel facial cleanser. U.S. Patent No. 5,466,456 to Glover discloses a facial cleanser.

Although the prior art teach inventions pertaining to cosmetic facial cleansers, they do not teach a facial cleanser that has the same ability to moisturize and cleanse. Moreover, the manner in which the present invention accomplishes the aforementioned functions is novel and unanticipated.

Consequently, the primary object of the present invention is to provide a facial cleanser and moisturizer.

A further object of the present invention is to provide a cosmetic facial cleanser composed of water subjected to electrolysis.

### SUMMARY OF THE INVENTION

The present invention comprises a multi-step process that yields high polarity water that is retained in human skin for longer periods of time and that has bactericidal effects. The process comprises a plurality of separate batch processes, wherein each successive process yields a product with greater moisturizing and bactericidal effects than the product yielded from the previous process. The process primarily comprises the passage of an electric current through water without the presence of electrolytes, which yields water that has a higher polarity and which is less effected by hydrogen bonding between water molecules. This results in smaller water molecule clusters normally caused by hydrogen banding. The reduced size of the clusters improves moisture penetration and retention in human skin. In addition, the increased polarity of the water is a harsh environment for bacteria.

These together with other objects of the invention are explained clearly in the claims annexed to and forming a part of this disclosure. For a better understanding of the invention, its operating advantages and the specific objects attained by its use, reference should be made to the accompanying drawings and descriptive matter in which there are illustrated preferred embodiments of the invention.

### BRIEF DESCRIPTION OF THE DRAWINGS

For a better understanding of the principle and nature of the present invention, reference should be made to the following detailed description taken in connection with the accompanying drawings in which:

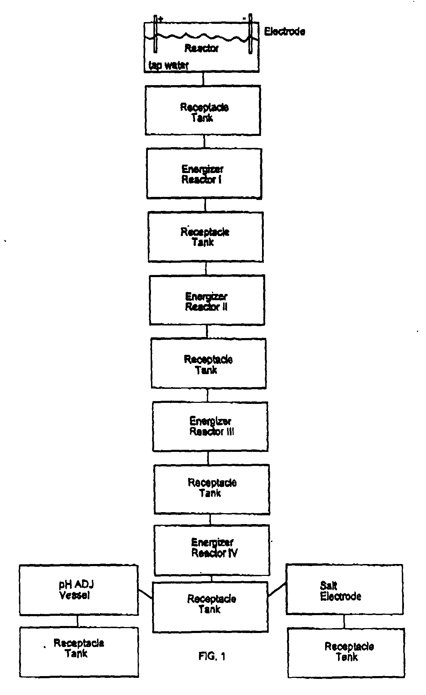

**FIG. 1** is a block flow diagram of the process for producing the present invention.

### DETAILED DESCRIPTION OF THE PREFERRED EMBODIMENT

Referring to **FIG. 1**, the present invention is a multi-step process that comprises a plurality of batch processes, wherein the product yielded from a step in the overall process is then successively used as the feed for the subsequent batch process.

The first batch process involves the passage of an electrical current through water. The water used is preferably filtered and is without electrolytes or contaminants, so as to maximize the oxygen content within the water, as water with dissolved contaminants like salt is unable to be fully saturated with oxygen. The voltage of the current is preferably between 50 and 100 volts. The process may use multiple cathodic and anodic leads, so as to maximize the effects of the current. In addition, stirrers may be employed in the reaction as well. This step yields a product with a lower viscosity.

The second batch process comprises an energizing reactor that comprises a batch process tank reactor with several far infrared light emitters. These emitters are positioned along the tank so as to maximize exposure to the water. The effluent yielded from the first batch process is then fed into the energizer reactor where the water is irradiated with far infrared light by the emitters. This irradiation by far infrared light also has the net effect of reducing the viscosity of the exposed water.

A plurality of these energizer reactors are placed in series, wherein the effluent from one energizer reactor serves as the feed for the subsequent energizer reactor down the process chain. Additional energizing reactors may be placed in the process chain, but considerable efficiency is lost with each subsequent reactor.

Effluent from the fourth energizer reactor may be fed to a pH adjusting vessel wherein the solution is introduced to basic compounds in order to reduce to pH of the solution, wherein the basic compounds are subsequently flushed or separated from the solution using conventional separating means. Effluent from the fourth energizer reactor may also be fed into a reactor with an active salt electrode. The resulting water is then used in the composition of a cosmetic facial cleanser. Other conventional cleansing agents may be added to the resulting water subjected to electrolyis.

## Claims

1. A process for producing a facial moisturizer and cleanser comprising:
a) filtration of water;
b) electrolysis of said water using a cathode and anode, and a current of voltage ranging between 50 to 100 volts;
c) irradiation of said water by far infrared light.

2. A process for producing a facial moisturizer and cleanser as mentioned in claim 1, wherein said water is irradiated by far infrared light of varying wavelengths.

3. A process for producing a facial moisturizer and cleanser as mentioned in claim 1, wherein pH of said water is adjusted.

4. A process for producing a facial moisturizer and cleanser as mentioned in claim 1, wherein said water is introduced to a salt electrode.

5. A process for producing a facial moisturizer and cleanser as mentioned in claim 1, wherein cleansing agents are added to said water.

6. A cosmetic cleanser or moisturizer comprising water processed to provide smaller clusters of water molecules of higher polarity than tap water.
